(19) Europäisches Patentamt
European Patent Office
Office européen des brevets

(11) Veröffentlichungsnummer: **0 045 982**
**B1**

# (12) EUROPÄISCHE PATENTSCHRIFT

(45) Veröffentlichungstag der Patentschrift: **15.02.84**

(51) Int. Cl.³: **C 07 F 5/00**, C 07 C 63/08, C 07 C 63/04, C 07 C 51/41, C 01 B 11/18

(21) Anmeldenummer: **81200488.5**

(22) Anmeldetag: **08.05.81**

(54) **Verfahren zur Herstellung von Arylthalliumcarboxylatperchloraten, 2-Carbonyloxiphenylthallium(III)-perchlorat und seine Herstellung und Weiterverarbeitung.**

(30) Priorität: **09.08.80 DE 3030273**

(43) Veröffentlichungstag der Anmeldung: **17.02.82 Patentblatt 82/7**

(45) Bekanntmachung des Hinweises auf die Patenterteilung: **15.02.84 Patentblatt 84/7**

(84) Benannte Vertragsstaaten: **DE FR GB IT NL**

(56) Entgegenhaltungen:
**BULLETIN OF THE CHEMICAL SOCIETY OF JAPAN, Band 44, Nr. 2, Februar 1971, Seiteb 545-550, K. ICHIKAWA et al.: "Reaction of Aromatics with Thallic Salts - Preparation of Arylthallic Compounds"**

(73) Patentinhaber: **Rütgerswerke Aktiengesellschaft, Mainzer Landstrasse 217, D-6000 Frankfurt a.Main 1 (DE)**

(72) Erfinder: **Knips, Ulrich, Dr., Werver Mark 174, D-4618 Kamen-Herren-Werve (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Arylthalliumcarboxylatperchloraten, 2-Carbonyloxiphenylthallium-(III)-perchlorat und seine Herstellung und Weiterverarbeitung

Die Erfindung betrifft ein Verfahren zur Herstellung von in hohem Masse isomerenfreien Arylthalliumcarboxylat-perchloraten durch Umsetzung von Thallium(III)-perchlorat, einer Carbonsäure und einem Aromaten in Wasser enthaltender Perchlorsäure nach Art einer Thallierungsreaktion. Diese Arylthalliumsalze werden als Zwischenprodukte benötigt, z.B. bei der Herstellung von Carbonsäurearylestern.

In Bull. Chem. Soc. Jap. 44, 545 (1971) ist die Darstellung von Arylthallium(acetat)-perchlorat-monohydraten der allgemeinen Formel $ArTl(OAc)(ClO_4)\cdot H_2O$ beschrieben, wobei Ar = Phenyl, Tolyl, Xylol oder Anisyl, OAc = Acetat bedeuten kann. Die Herstellung erfolgt in essigsaurer Lösung aus etwa äquimolaren Mengen $Tl(OAc)_3$ und Perchlorsäure mit einem Überschuss an Aromaten. Die Essigsäuremenge entspricht einem mindestens 20fachen molaren Überschuss, bezogen auf das Thalliumsalz. Für die Thallierung von Toluol wird die erforderliche Reaktionstemperatur (und -zeit) mit 80°C (8,5 h), beim Benzol mit 90°C (6 h) und bei den Xylolen mit ca. 110°C (2 h) angegeben. Lediglich Anisol ist bei milderen Bedingungen (35°C) innerhalb einer Stunde in 44%iger Ausbeute umsetzbar. Die Ausbeuten an Arylthalliumsalzen in den anderen Fällen werden mit 54,9% bei Benzol, 51,3% bei m-Xylol, 38,4% bei o-Xylol, 22,6% bei p-Xylol und 67,0–73,1% bei Toluol angegeben. Im experimentellen Teil der Arbeit finden sich jedoch beträchtlich niedrigere Ausbeuten an isolierbarer Substanz bei gleichzeitiger Erwähnung beträchtlicher Mengen an Nebenprodukten. Allerdings sind die Reaktionsbedingungen mit den bei der Auflistung der Ausbeuten gemachten Angaben nicht identisch. Eigene Versuche zeigten jedoch, dass auch bei Einhaltung der entsprechenden Bedingungen Produkte mit den besseren Ausbeuten nicht isolierbar waren, sich diesen Angaben also wahrscheinlich nur auf in Lösung nachweisbare Mengen des Arylthalliumsalzes beziehen. Gegen hohe isolierbare Ausbeuten sprechen ausserdem die dunkle Verfärbung der Lösung und die in erheblichem Masse zu beobachtende Bildung von $Tl^{I}ClO_4$ als Folge von Zersetzungsreaktionen.

Der vorliegenden Erfindung lag die Aufgabe zugrunde, Arylthalliumcarboxylat-perchlorate in hohen Ausbeuten bei kurzen Reaktionszeiten darzustellen. Darüberhinaus sollten die Bedingungen so schonend sein, dass Nebenreaktionen weitgehend zurückgedrängt werden und nahe bei 100% liegende Isomerenreinheit der Organolthalliumsalze erreicht wird. Diese Kriterien stellen wesentliche Voraussetzungen für die Realisierung eines technischen Verfahrens dar.

Die gestellte Aufgabe wird erfindungsgemäss dadurch gelöst, dass die Reaktionspartner in den auf das Thallium(III)-perchlorat bezogenen molaren Verhältnissen von 1 bis 6 für die Carbonsäure, von 1 bis 6 für die aromatische Verbindung, und von 2 bis 15 für die Perchlorsäure, die 30–45% Wasser enthalten kann, bei normalem Druck und Temperaturen von 0–80°C zur Reaktion gebracht und die nach einigen Minuten ausfallenden Monoarylthalliumcarboxylatperchlorate in üblicher Weise abgetrennt werden, als aromatische Verbindungen kommen Benzol, Toluol, die Xylole, oder die Butylbenzole, als Carbonsäuren Ameisen-, Essig-, Propion- oder die n- oder iso-Buttersäure in Betracht.

Thallium(III)-perchlorat kann in fester Form zugegeben werden oder in der Reaktionsmischung durch Auflösen von $Tl_2O_3$ oder geeigneter Thalliumsalze wie Thalliumacetat hergestellt werden. Vorteilhafter ist die Darstellung des $Tl(ClO_4)_3$ aus $TlClO_4$ in 55–70%iger Perchlorsäure durch anodische Oxidation. Diese Verfahrensführung erlaubt es, die bei allen Umsetzungen von Arylthalliumsalzen anfallenden Thallium(I)-salze ohne zusätzlichen Hilfsstoffeinsatz zu rezyklisieren. Die nach Beendigung der Oxidation anfallende Anodenlösung ist nach Zugabe von Carbonsäure direkt für die Aromatenthallierung verwendbar.

Die Arylthalliumsalze fallen aus den Reaktionsgemischen nach wenigen Minuten bei Raumtemperatur aus. Die Reaktionszeiten werden durch Erhöhung der Carbonsäure- und/oder Wasserkonzentration verlängert. Bei grösseren als den oben angegebenen Mengen dieser Stoffe ist bei Raumtemperatur keine Isolierung des Arylthalliumsalzes mehr möglich, bzw. wird die Reaktion gänzlich unterbunden.

Die ausgeschiedenen Salze werden nach bekannten Methoden von der Mutterlauge abgetrennt, die erneut nach Eintragen von $Tl(ClO_4)_3$ zur Reaktion Verwendung finden kann. Vorteilhaft wird gemäss Anspruch 3 die Mutterlauge mit $TlClO_4$ versetzt und einer anodischen Oxidation unterworfen, nach deren Abschluss eine direkt verwendbare Reaktionslösung resultiert. Es ist Sorge zu tragen, dass die Arylthalliumsalze möglichst vollständig von anhaftender Mutterlauge befreit werden, da schon geringe Säuremengen eine nachfolgende gewünschte Umsetzung zum Arylester negativ beeinflussen.

Die Thallierungsreaktion wird im Temperaturbereich von 0–80°C, vorzugsweise jedoch bei Raumtemperatur durchgeführt, da bei höheren Temperaturen der Anteil an Isomeren zunimmt. Hinsichtlich der elektrophilen Substitution desaktivierte Aromaten, wie Benzoesäure, bedingen zur Verkürzung der Reaktionszeiten Temperaturen von 40–80°C, vorzugsweise 60°C. Die dargestellten Salze sind ohne Umkristallisation zu mehr als 95% isomerenrein und enthalten nur noch geringe Mengen des eingesetzten Aromaten, der Carbonsäure oder der Perchlorsäure, so dass eine nachfolgende Reinigung dieser Salze für die Umsetzung zu den Estern oder Biarylen entfallen kann.

Die Ausbeuten an isolierten Salzen betragen 80–90% und sind bei Rückführung der Mutterlaugen noch steigerbar. Die Arylthalliumsalze haben

eine Reinheit von 90–95% bei der angesprochenen hohen Isomerenreinheit. Durch Umkristallisieren aus Eisessig lassen sich erforderlichenfalls die Reinsubstanzen herstellen.

Es wurde gefunden, dass aromatische Carbonsäuren sowohl als aromatische Komponente, wie als Carbonsäure reagieren, so dass der Zusatz von zusätzlicher Carbonsäure - es sei denn zur Förderung der Löslichkeit - unterbleiben kann. Erfindungsgemäss wurde so das Benzoesäure-Thallierungsprodukt gemäss der Formel

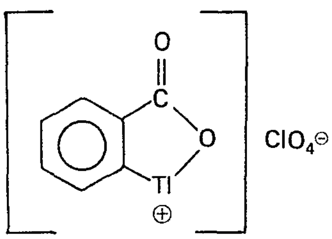

hergestellt.

Den Beweis für die Struktur liefern das NMR-Spektrum und eine Elementaranalyse des entsprechenden Hydroxids. Das alkohollösliche Phenylcarbonyloxithallium(III)-perchlorat ermöglicht überraschenderweise die einfache Herstellung von 2-Acylsalicylaten in quantitativer Ausbeute. In wässriger Lösung erfolgt nicht eine erwartete Aufspaltung des thalliumorganischen Ringes, sondern eine Ausfällung des entsprechenden Hydroxids, das bemerkenswert stabil ist. Das Hydroxid ist in organischen Lösungsmitteln unlöslich, löst sich jedoch in konzentrierten Lösungen starker Säuren, so dass dadurch die Darstellung einer Vielzahl von Salzen der komplexen Arylthallium(III)-carboxylat-kationen ermöglicht wird. Es zeigt amphoteres Verhalten, da es auch in starken Laugen löslich ist. Durch Umsetzung mit Acylierungsmitteln in wässrigem Medium entstehen in quantitativer Reaktion Acylsalicylsäuren als vollkommen reine Endprodukte.

Die Verwendung der erfindungsgemäss dargestellten Arylthalliumsalze ist nicht auf die Nutzung zur Darstellung von Carbonsäurearylestern und Biarylen beschränkt, sondern grundsätzlich für jede bekannte Reaktion von Monoarylthalliumsalzen, wie die Darstellung von Aryljodiden, verwendbar.

Beispiel 1

Eine Lösung von 507 g (1 Mol) $Tl(ClO_4)_3$ in 560 g (3 Mol) 55%iger Perchlorsäure und 60 g (1 Mol) Essigsäure wird unter kräftigem Rühren bei Raumtemperatur mit 100 g (1,1 Mol) Toluol versetzt. Nach wenigen Minuten fällt ein farbloser Niederschlag von p-Tolylthalliumacetat-perchlorat-hydrat aus, der bei nicht ausreichendem Rühren zum Erstarren der Reaktionsmischung führen kann. Das ausgefallene Salz wird abzentrifugiert und mehrfach mit Toluol nachgewaschen. Die Ausbeute an fast trockenem, farblosem, zu 98% isomerenreinem Organothalliumsalz beträgt 425 g (=90% d.Th.).

Beispiel 2

Eine Aufschlämmung von 200 g (0,66 Mol) $TlClO_4$ in 500 g (3 Mol) 60%iger $HClO_4$ wird der anodischen Oxidation unterworfen. Die so erhaltene $Tl^{3+}$-Lösung (etwa 0,6 Mol) wird mit 40 g (0,66 Mol) Essigsäure und 60 g (0,66 Mol) Toluol versetzt und gemäss Beispiel 1 aufgearbeitet.

Beispiele 3–11

Nach Beispiel 1 und 2 hergestellte $Tl^{3+}$-Lösungen in $HClO_4$ werden mit äquimolaren Mengen Essigsäure und den in der folgenden Tabelle beispielhaft aufgeführten Aromaten versetzt. Die beobachteten Phänomene und die Aufarbeitung entsprechen dem in den Beispielen 1 und 2 Gesagten.

| Nr. | Aromat | Ausbeute % | Arylrest im Arylthalliumacetatperchlorat |
|---|---|---|---|
| 3 | Benzol | 83 | Phenyl- |
| 4 | o-Xylol | 85 | 3,4-Dimetylphenyl |
| 5 | m-Xylol | 88 | 2,4-Dimethylphenyl-* |
| 6 | p-Xylol | 84 | 2,5-Dimethylphenyl- |
| 7 | Äthylbenzol | 81 | 4-Äthylphenyl- |
| 8 | n-Butylbenzol | 85 | 4-n-Butylphenyl- |
| 9 | sec.-Butylbenzol | 87 | 4-sec.-Butylphenyl- |
| 10 | iso-Butylbenzol | 83 | 4-iso-Butylphenyl- |
| 11 | tert.-Butylbenzol | 81 | 4-tert.-Butylphenyl- |

Die Reaktionszeiten betragen in allen Fällen 10–15 Minuten.

* bildet kein Hydrat

Beispiele 12–15

Gemäss der Beispiele 1 und 2 hergestellte $Tl^{3+}$-Lösungen werden mit äquimolaren Mengen einer Carbonsäure versetzt. Nach Zugabe einer äquimolaren Menge Toluol entstehen Salze, die entsprechend den vorherigen Beispielen gewonnen werden können.

| Nr. | Carbonsäure | Ausbeute % | Endprodukt |
|---|---|---|---|
| 12 | Propionsäure | 83 | p-Tolylthalliumpropionat-perchlorat |
| 13 | Ameisensäure | 81 | p-Tolylthalliumformiat-perchlorat |
| 14 | n-Buttersäure | 85 | p-Tolylthalliumbutyrat-perchlorat |
| 15 | iso-Buttersäure | 83 | p-Tolylthalliumisobutyrat-perchlorat |

Die Reaktionszeiten betragen in allen Fällen 10–15 Minuten.

Beispiel 16

122 g Benzoesäure werden in eine Lösung von 503 g $Tl(ClO_4)_3$ und 60 g Essigsäure in 500 g 55%iger Perchlorsäure eingetragen und bei 60°C 5 Stunden lang kräftig verrührt. Nach dem Abzentrifugieren des Niederschlags wird aus diesem mit Äther die überschüssige Benzoesäure extrahiert und zur Abtrennung geringer Mengen $TlClO_4$ mit Äthylalkohol aufgenommen. Nach Abdampfen des Lösungsmittels wird «komplexes» 2-Carbonyloxiphenylthallium(III)-perchlorat isomerenrein, acetat- und benzoatfrei (nach NMR) in 63%iger Ausbeute erhalten.

Beispiel 17

Die nach Beispiel 16 erhaltene Lösung des 2-Carbonyloxiphenylthallium(III)-perchlorats in Alkohol wird mit Wasser versetzt und die Vollständigkeit der Fällung mit wenig verdünnter Natronlauge überprüft. Der Niederschlag wird abgenutscht, mit Wasser gewaschen und getrocknet. Ausbeute: 205 g (60% d. Th.) 2-Carbonyloxiphenylthallium(III)-hydroxid.

Analysendaten für $C_7H_5O_3Tl$:

| | | | | |
|---|---|---|---|---|
| Gef.: | C 24,91% | H 1,64% | O 13,96% | Tl 59,19% |
| Ber.: | C 24,62% | H 1,48% | O 14,06% | Tl 59,85% |

**Patentansprüche**

1. Verfahren zur Herstellung von Arylthallium(III)-carboxylatperchloraten, dadurch gekennzeichnet, dass man 1 Mol Thallium(III)-perchlorat, 1 bis 6 Mol einer Carbonsäure, 1 bis 6 Mol einer aromatischen Verbindung und 2 bis 15 Mol Perchlorsäure, die 30–45% Wasser enthalten kann, bei normalem Druck und Temperatur von 0–80°C zur Reaktion bringt, wobei als aromatische Verbindungen Benzol, Toluol, die Xylole oder die Butylbenzole und als Carbonsäuren Ameisen-, Essig-, Propion- oder n- oder iso-Buttersäure in Betracht kommen.

2. Verfahren nach Anspruch 1, dadurch gekennzeichnet, dass die Reaktion bei Raumtemperatur durchgeführt wird.

3. Verfahren nach den Ansprüchen 1 und 2, dadurch gekennzeichnet, dass Thallium(III)-perchlorat aus Thallium(I)-perchlorat, -hydroxid oder -carboxylaten in Perchlorsäure durch anodische Oxidation dargestellt und diese Lösung nach Versetzen mit der Carbonsäure und dem aromatischen Kohlenwasserstoff direkt als Reaktionslösung benutzt wird.

4. Phenylcarbonyloxithallium(III)-perchlorat der Struktur

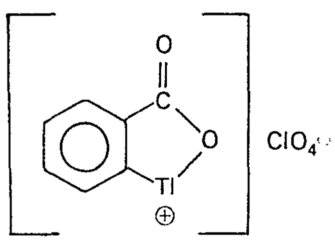

5. Verfahren zur Herstellung von Phenylcarbonyloxithallium(III)-perchlorat nach Anspruch 4, dadurch gekennzeichnet, dass Thallium(III)-perchlorat mit Benzoesäure im molaren Verhältnis von 1:1–6 in 55–70%iger Perchlorsäure bei normalem Druck und Temperaturen von 40 bis 80°C zur Reaktion gebracht wird.

6. Verfahren zur Herstellung von Phenylcarbonyloxithallium(III)-perchlorat gemäss der Ansprüche 4 und 5, dadurch gekennzeichnet, dass die Reaktion bei 60°C durchgeführt wird.

7. Verfahren zur Herstellung von Phenylcarbonyloxithallium(III)-hydroxid der Struktur

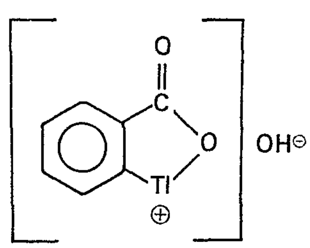

dadurch gekennzeichnet, dass man das Phenylcarbonyloxithallium(III)-perchlorat in einem Lösungsmittel löst und mit Wasser umsetzt.

**Claims**

1. A process for the preparation fo arylthallium(III)-carboxylate perchlorates comprising reacting 1 mole thallium(III)-perchlorate with 1 to 6 moles of a carboxylic acid, 1 to 6 moles of an aromatic compound and 2 to 15 moles of perchloric acid containing 3 to 45% by weight of water at normal pressure and temperature of 0 to 80°C wherein as aromatic compounds benzene, toluene, the xylenes or the butylbenzenes and as carboxylic acids formic-, acetic-, propionic-or n- or iso-butyric acid are used.

2. A process of claim 1 wherein the reaction temperature is room temperaure.

3. A process of claims 1 and 2 wherein thallium(III)-perchlorate is prepared by anodic oxidation of a perchloric acid solution of a thallium(I)-perchlorate, -hydroxide or -carboxylate and wherein this solution is directly used as reaction solution after adding the carboxylic acid and the aromatic compound.

4. Phenylcarbonylthallium(III)-perchlorate of the formula

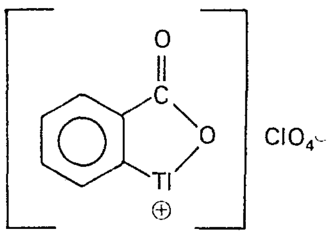

5. A process for the preparation of phenylcarbonylthallium(III)-perchlorate of claim 4 comprising reacting thallium(III)-perchlorate with benzoic acid in a molar ratio of 1:1–6 in 55 to 70% perchloric acid at normal pressure and temperatures of 40 to 80°C.

6. A process for the praparation of phenylcarbonyl thallium(III)-perchlorate of claims 4 and 5 wherein the reaction temperature is 60°C.

7. A process for the preparation of phenylcarbonyl thallium(III)-hydroxide of the formula

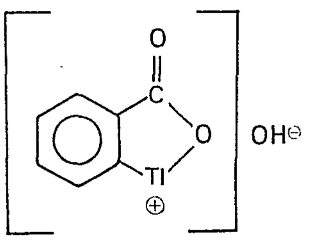

wherein the phenylcarbonyl thallium(III)-perchlorate is solved in a solvent and reacted with water.

**Revendications**

1. Procédé de préparation de carboxylate-perchlorates de arylthallium(III) par réaction de perchlorate de thallium(III), d'un acide carboxylique et d'un composé aromatique dans de l'acide perchlorique contenant de l'eau, procédé caractérisé en ce qu'on fait réagir les partenaires réactionnels dans les proportions molaires suivantes relativement au perchlorate de thallium(III): de 1 à 6 pour l'acide carboxylique, 1 à 6 pour le composé aromatique et 2 à 15 pour l'acide perchlorique, lequel peut contenir de 30 à 45% d'eau, sous la pression normale et à des températures de 0 à 80°C, où comme composé aromatique de benzene, de toluene, des xyloles ou des benzenes de butyle et comme acide carboxylique d'acide formique, acetique, propionique ou n- ou iso-butylique sont utilisées.

2. Procédé selon la revendication 1, caractérisé en ce que la réaction est effectuée à la température ambiante.

3. Procédé selon l'une des revendications 1 et 2, caractérisé en ce qu'on prépare le perchlorate de thallium(III) à partir du perchlorate, de l'hydroxyde ou de carboxylates de thallium(I) dans de l'acide perchlorique par oxydation anodique et on utilise directement cette solution, après y avoir ajouté l'acide carboxylique et hydrocarbure aromatique, comme solution réactionnelle.

4. Phenylcarboxylate-perchlorate de thallium(III) qui répond à la formule suivante:

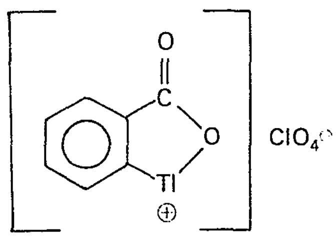

5. Procédé de préparation de phenylcarboxylate-perchlorate de thallium(III) selon la revendication 4, procédé caractérisé en ce qu'on réagit le perchlorate de thallium(III) avec acide benzoïque dans un rapport molaire compris entre 1:1 et 1:6, dans le l'acide perchlorique d'une concentration comprise entre 55 et 70%, sous la pression normale et à des températures de 40 à 80°C.

6. Procédé de préparation du phenylcarboxylate-perchlorate de thallium(III) selon l'une des revendications 4 et 5, procédé caractérisé en ce qu'on effectue la réaction à 60°C.

7. Procédé de préparation de phenylcarboxylate-hydroxide de thallium(III) qui repond à la formule suivante:

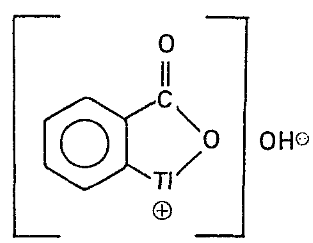

caractérisé en ce qu'on dissoude le phenylcarboxylate-perchlorate de thallium(III) dans un solvent et qu'on le fait réagir avec de l'eau.

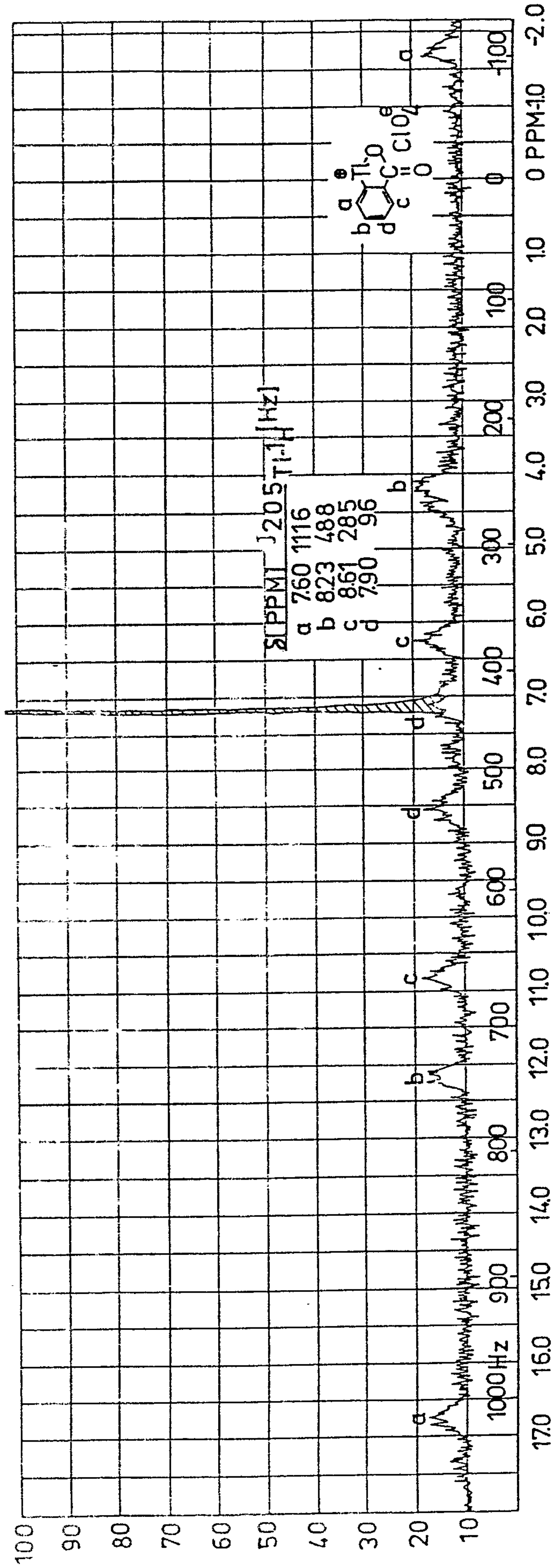
δ [PPM]   J205Tl-1H [Hz]
a 7.60 1116
b 8.23 488
c 8.61 285
d 7.90 96
0 PPM -1.0
1000 Hz